(19) Europäisches Patentamt
European Patent Office
Office européen des brevets

(11) **EP 3 024 869 B1**

(12) **EUROPÄISCHE PATENTSCHRIFT**

(45) Veröffentlichungstag und Bekanntmachung des Hinweises auf die Patenterteilung:
**06.05.2020 Patentblatt 2020/19**

(21) Anmeldenummer: **14741319.9**

(22) Anmeldetag: **21.07.2014**

(51) Int Cl.:
*C08G 18/78* *(2006.01)*     *C07D 273/04* *(2006.01)*
*C08G 18/79* *(2006.01)*     *C08G 18/02* *(2006.01)*
*C08G 18/09* *(2006.01)*

(86) Internationale Anmeldenummer:
**PCT/EP2014/065576**

(87) Internationale Veröffentlichungsnummer:
**WO 2015/011069 (29.01.2015 Gazette 2015/04)**

(54) **VERFAHREN ZUR HERSTELLUNG VON POLYISOCYANATEN UND DEREN VERWENDUNG**

METHOD FOR MANUFACTURING POLYISOCYANATES AND THEIR APPLICATION

PROCÉDÉ DE FABRICATION DE POLYISOCYANATES ET LEUR UTILISATION

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **25.07.2013 EP 13177979**

(43) Veröffentlichungstag der Anmeldung:
**01.06.2016 Patentblatt 2016/22**

(73) Patentinhaber: **Covestro Deutschland AG**
**51373 Leverkusen (DE)**

(72) Erfinder:
• **RICHTER, Frank**
  **51373 Leverkusen (DE)**
• **HALPAAP, Reihard**
  **51519 Odenthal (DE)**

(74) Vertreter: **Levpat**
  **c/o Covestro AG**
  **Gebäude 4825**
  **51365 Leverkusen (DE)**

(56) Entgegenhaltungen:
**EP-A1- 0 339 396    EP-A1- 0 962 455**
**EP-A1- 1 645 577    EP-A2- 0 379 914**
**EP-A2- 0 447 074**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

**Beschreibung**

Die vorliegende Erfindung betrifft ein Verfahren zur Herstellung von Iminooxadiazindiongruppen enthaltenden Polyisocyanaten, bei dem mindestens ein monomeres Di- und/oder Triisocyanat, in Gegenwart von

a) mindestens einem Katalysator,
b) mindestens einem Additiv (A) mit einer relativen Permittivität bei 18 °C bis 30 °C von wenigstens 4,0,
c) gegebenenfalls weiteren, von A verschiedenen Additiven oligomerisiert wird. Die Erfindung betrifft ferner ein Reaktionssystem zur Herstellung von Iminooxadiazindiongruppen enthaltenden Polyisocyanaten sowie die Verwendung eines Additivs (A) mit einer relativen Permittivität bei 18 °C bis 30 °C von wenigstens 4,0 zur Herstellung von Iminooxadiazindiongruppen enthaltenden Polyisocyanaten durch katalysierte Modifizierung monomerer Di- und/oder Triisocyanate.

Die Oligo- bzw. Polymerisierung von Isocyanaten, hier zusammenfassend Modifizierung genannt, ist seit langem bekannt. Enthalten die modifizierten Polyisocyanate freie NCO-Gruppen, die ggf. auch mit Blockierungsmitteln vorübergehend desaktiviert worden sein können, sind sie außerordentlich hochwertige Ausgangsstoffe für die Herstellung einer Vielzahl von Polyurethan-Kunststoffen und Beschichtungsmitteln.
Es haben sich eine Reihe technischer Verfahren zur Isocyanatmodifizierung etabliert, wobei man in der Regel das zu modifizierende Isocyanat, meist ein Diisocyanat, durch Zusatz von Katalysatoren umsetzt und diese anschließend, wenn der gewünschte Umsatzgrad des zu modifizierenden Isocyanates erreicht ist, durch geeignete Maßnahmen unwirksam macht (deaktiviert) und das erhaltene Polyisocyanat in der Regel vom nicht umgesetzten Monomer abtrennt. Eine Zusammenstellung dieser Verfahren des Standes der Technik findet sich in H. J. Laas et al., J. Prakt. Chem. 1994, 336, 185 ff.
Eine spezielle Form der Isocyanatmodifizierung die zu Produkten mit einem hohen Anteil an Iminooxadiazindiongruppen (asymmetrischen Isocyanat-Trimeren), neben den lange bekannten Isocyanuratstrukturen (symmetrischen Isocyanat-Trimeren, bisher vereinfachend häufig nur als "Trimere" bezeichnet) in den Verfahrensprodukten führt, wird u.a. in EP 962 455 A1, EP 962 454 A1, EP 896 009 A1, EP 798 299 A1, EP 447 074 A1, EP 379 914 A1, EP 339 396 A1, EP 315 692 A1, EP 295 926 A1 sowie EP 235 388 A1 beschrieben. Als Katalysatoren hierfür haben sich u.a. (Hydrogenpoly)fluoride bewährt.
Ein Nachteil der bekannten Verfahren des Standes der Technik ist, dass sich die als Katalysator verwendeten Spezies teilweise unter Bildung störender Nebenprodukte zersetzen, was sich bei Verfahren, in denen mit (Hydrogenpoly)fluoriden operiert wird, die ein quaternäres P-Atom als Gegenion enthalten, durch einen sukzessiv ansteigenden Phosphorgehalt des - in der Regel destillativ - wiedergewonnenen Monomers (Recyclat) äußert. Zwar lassen sich derartig verunreinigte Recyclate aufreinigen, wie beispielsweise in EP 1 939 171 A1 beschrieben, jedoch ist eine derartige Vorgehensweise mit zusätzlichem Aufwand verbunden, den es zu vermeiden gilt.
Der Erfindung lag daher die Aufgabe zugrunde, ein Verfahren zur Herstellung hoch Iminooxadiazindionngruppen enthaltender Polyisocyanate zur Verfügung zu stellen, das nicht mit den vorgenannten Nachteilen behaftet ist: die Katalysatoren sollten eine bessere Stabilität im Isocyanatmilieu aufweisen und nicht bzw. im Vergleich mit Systemen des Standes der Technik weniger zur Zersetzung unter Bildung störender Nebenkomponenten neigen, die sich in den Verfahrensprodukten, insbesondere dem Recyclat, anreichern können. Insbesondere soll das Verfahren ohne zusätzliche Aufreinigung auskommen.
Diese Aufgabe wurde gelöst durch ein Verfahren zur Herstellung von Iminooxadiazindiongruppen enthaltenden Polyisocyanaten, bei dem mindestens ein monomeres Di- und/oder Triisocyanat, in Gegenwart von

a) mindestens einem Katalysator,
b) mindestens einem Additiv mit einer relativen Permittivität bei 18 °C bis 30 °C von wenigstens 4,0,
c) gegebenenfalls weiteren Additiven

oligomerisiert wird, wobei 1 bis 50 Gew.-% an Additiv (A), bezogen auf die Masse des monomeren Di- und/oder Triisocyanats, eingesetzt werden.
Der vorliegenden Erfindung liegt die Erkenntnis zugrunde, dass durch das erfindungsgemäß eingesetzte Additiv entsprechender relativer Permittivität hoch Iminooxadiazindionngruppen haltige Polyisocyanate erzeugen lassen, ohne dass hierfür zusätzliche Aufreinigungsschritte erforderlich wären. Keiner der eingangs genannten Schriften des Standes der Technik ist zu entnehmen, dass die zur Iminooxadiazindionbildung bevorzugten Katalysatoren des Standes der Technik in Gegenwart der vorgenannten Additive eine signifikante Stabilisierung im Isocyanatmilieu erfahren. Da es sich bei den Diisocyanate selbst bereits um polare Verbindungen handelt war zudem auch nicht zu erwarten, dass der Zusatz weiterer polarer Substanzen zum Reaktionsgemisch hier einen signifikanten Einfluss ausüben könnte.
Die relative Permittivität bei 18 °C bis 30 °C wird im Sinne der vorliegenden Erfindung bestimmt durch das Verhältnis der Kapazitäten eines Kondensators mit der Substanz einerseits und Vakuum andererseits als Dielektrikum bei einer Messfrequenz von 50 Hz. Dies lässt sich durch den allgemein bekannten folgenden Zusammenhang ausdrücken:

$$\varepsilon_r = \frac{\varepsilon}{\varepsilon_0}$$

mit der relative Permittivität $\varepsilon_r$, gemessene Permittivität der Substanz $\varepsilon$ und zu der elektrischen Feldkonstante $\varepsilon_0$. Hierbei bedeutet der genannte Temperaturbereich von 18 °C bis 30 °C, dass die Substanz bei einer beliebigen Temperatur in diesem Bereich die angegebene relative Permittivität aufweist. Besitzt ein Additiv also beispielsweise bei 30 °C eine relative Permittivität von 4,0, bei 20 °C hingegen ein geringere relative Permittivität, so erfüllt dieses Additiv dennoch die erfindungsgemäße Definition einer relativen Permittivität bei 18 °C bis 30 °C von wenigstens 4,0. Selbiges gilt analog für die weiter unten definierten Vorzugsbereiche hinsichtlich der relativen Permittivität. So besitzt beispielsweise Gamma-Valerolacton eine relative Permittivität von 36,9 bei 20 °C und von 34,5 bei 30 °C.

Die folgende Tabelle vermittelt einen Überblick über typische Werte der relativen Permittivitäten verschiedener Stoffe, jeweils gemessen bei 50 Hz:

| Lösungsmittel | relative Permittivität bei | °C |
|---|---|---|
| n-Hexan | 1,9 | 25 |
| n- Heptan | 2,0 | 25 |
| Cyclohexan | 2,0 | 20 |
| Isoeicosan | 2,1 | 25 |
| 1,4-Dioxan | 2,2 | 25 |
| Tetrachlorkohlenstoff | 2,2 | 20 |
| Benzol | 2,3 | 25 |
| Tetrachlorethen | 2,3 | 25 |
| Toluol | 2,4 | 25 |
| Triethylamin | 2,4 | 25 |
| Schwefelkohlenstoff | 2,6 | 20 |
| Trichlorethen | 3,4 | 16 |
| Anisol | 4,3 | 25 |
| Dibutylether | 4,3 | 20 |
| Diethylether | 4,3 | 20 |
| Chloroform | 4,8 | 20 |
| Brombenzol | 5,4 | 25 |
| Chlorbenzol | 5,6 | 25 |
| Piperidin | 5,8 | 20 |
| Essigsäureethylester | 6,0 | 25 |
| Eisessig | 6,2 | 20 |
| Anilin | 6,9 | 20 |
| Ethylenglycoldimethylether | 7,2 | 25 |
| Triethylenglycoldimethylether (Triglyme) | 7,5 | 25 |
| 1,1,1-Trichlorethan | 7,5 | 20 |
| T etrahydro furan | 7,6 | 25 |

(fortgesetzt)

| Lösungsmittel | relative Permittivität bei | °C |
|---|---|---|
| Diethylenglycol | 7,7 | 25 |
| Methylenchlorid | 8,9 | 25 |
| Chinolin | 9,0 | 25 |
| Ethylendichlorid | 10,4 | 25 |
| Pyridin | 12,4 | 21 |
| 2-Methyl-2-propanol (tert-Butanol) | 12,5 | 25 |
| 3-Methyl-1-butanol (Isoamylalkohol) | 14,7 | 25 |
| 1 -Butanol | 17,5 | 25 |
| Methylethylketon (Butanon) | 18,5 | 20 |
| 2-Propanol (Isopropylalkohol) | 19,9 | 25 |
| Propanol | 20,3 | 25 |
| Essigsäureanhydrid | 20,7 | 19 |
| Aceton | 20,7 | 25 |
| Triethylenglycol | 23,7 | 20 |
| Ethanol | 24,6 | 25 |
| Benzonitril | 25,2 | 25 |
| Adiponitril | 30,0 | 18 |
| N-Methyl-2-pyrrolidon (NMP) | 32,2 | 25 |
| Methanol | 32,7 | 25 |
| Nitrobenzol | 34,8 | 25 |
| Nitromethan | 35,9 | 30 |
| Gamma-V alerolacton | 36,9 | 20 |
| Dimethylformamid | 37,0 | 25 |
| Acetonitril | 37,5 | 20 |
| Ethylenglycol | 37,7 | 25 |
| Dimethylacetamid | 37,8 | 25 |
| $\gamma$-Butyrolacton | 39,1 | 25 |
| Sulfolan | 43,3 | 30 |
| Dimethylsulfoxid | 46,7 | 25 |
| Propylencarbonat (4-Methyl-1,3-dioxol-2-on) | 65,1 | 25 |
| Wasser | 78,4 | 25 |
| Formamid | 111,0 | 20 |
| N-Methylformamid | 182,4 | 25 |

Nach einer Weiterbildung des erfindungsgemäßen Verfahrens wird dem monomeren Di- und/oder Triisocyanat das Additiv (A) vor dem Inkontaktbringen mit dem Katalysator zugesetzt. Hierzu wird das erfindungswesentliche Additiv dem/den zu modifizierenden Monomeren beigemischt.

In einer alternativen Ausführungsform wird das Additiv (A) dem Katalysator. bzw. der Katalysatorlösung beigemischt.

In vorteilhafter Ausgestaltung des erfindungsgemäßen Verfahrens weist das Additiv (A) eine relative Permittivität bei 18 °C bis 30 °C von wenigstens 8,0 auf, bevorzugt von wenigstens 20,0, besonders bevorzugt von wenigstens 30,0 oder gar wenigstens 35,0. Als Additiv (A) eignen sich beispielsweise Nitrile, Carbonate und/oder cyclische Lactone. Das Additiv (A) ist insbesondere ausgewählt aus der Gruppe umfassend Acetonitril, Adiponitril, Ethylencarbonat, Propylencarbonat und gamma-Valerolacton.

Nach einer bevorzugten Ausführungsform des erfindungsgemäßen Verfahrens werden bevorzugt 2 bis 30 Gew.-%, bezogen auf die Masse des monomeren Di- und/oder Triisocyanats eingesetzt, besonders bevorzugt 2 bis 20 Gew.-%. Technisch vorteilhaft ist eine möglichst niedrige Additivmenge um einerseits die Raum-Zeit-Ausbeute an Polyisocyanatharz möglichst hoch und den Katalysatorbedarf möglichst niedrig zu gestalten. Aber selbst bei Zusatz von 20 Gew.-% Acetonitril (die relative Permittivität $\varepsilon_r$ bei 20°C beträgt 37,5, s. Beispiel 2) liegt der Mehrbedarf an Katalysator noch durchaus im technisch akzeptablen Bereich bei deutlich reduzierter Katalysatorzersetzung.

Im Rahmen des erfindungsgemäßen Verfahrens können im Prinzip alle bekannten Isocyanate eingesetzt werden. Bevorzugt werden Di- und/oder Triisocyanate mit aliphatisch, cycloaliphatisch, araliphatisch und/oder aromatisch gebundenen Isocyanatgruppen einzeln oder in beliebigen Abmischungen untereinander eingesetzt. Hierbei ist es belanglos, nach welchen Verfahren die vorstehend genannten (Poly)isocyanate generiert werden, d.h. mit oder ohne Verwendung von Phosgen. Insbesondere seien genannt: Hexamethylendiisocyanat (HDI), 2-Methylpentan-1,5-diisocyanat, 2,4,4-Trimethyl-1,6-hexandiisocyanat, 2,2,4-Trimethyl-1,6-hexandiisocyanat, 4-Isocyanatomethyl-1,8-octandiisocyanat, 3(4)-Isocyanatomethyl-1-methylcyclohexylisocyanat (IMCI), Isophorondiisocyanat (IPDI), 1,3- sowie 1,4-Bis(isocyanatomethyl)benzen (XDI), 1,3- sowie 1,4-Bis(isocyanatomethyl)cyclohexan (H6XDI), 2,4- sowie 2,6-Toluylendiisocyanat (TDI), Bis(4-isocyanatophenyl)methan (4,4'MDI), 4-Isocyanatophenyl-2-isocyanatophenylmethan (2,4'MDI) sowie mehrkernige Produkte, die durch Formaldehyd-Anilin-Polykondensation und anschließende Überführung der resultierenden (Poly)amine in die entsprechenden (Poly)isocyanate zugänglich sind (Poymer-MDI). Bevorzugt werden aliphatische Di- und/oder Triisocyanate, besonders bevorzugt aliphatische Diisocyanate eingesetzt. Ganz besonders bevorzugt wird Hexamethylendiisocyanat (HDI), 2-Methylpentan-1,5-diisocyanat, 2,4,4-Trimethyl-1,6-hexandiisocyanat, 2,2,4-Trimethyl-1,6-hexandiisocyanat und/oder 4-Isocyanatomethyl-1,8-octandiisocyanat eingesetzt, noch mehr bevorzugt ist HDI.

Als Katalysatoren kommen prinzipiell alle für diesen Zweck vorbeschriebenen Verbindungen des Standes der Technik als solche oder in Lösung in Frage. Besonders geeignet sind salzartig aufgebaute Substanzen mit Kationen, die für eine gute Löslichkeit im Isocyanatmedium sorgen, besonders Tetraorganyl-ammoniumsalze und -phosphoniumsalze, mit Anionen, ausgewählt aus der Gruppe RfCR$_1$R$_2$COOH, wobei Rf für einen geradkettigen oder verzweigten Perfluoralkylrest und $R_1$ und $R_2$ unabhängig voneinander für H oder geradkettige oder verzweigte Organylreste stehen, Fluorid (F$^-$), Di- und/oder Poly(hydrogen)fluoride ([F$^-$ x HF)$_m$]), wobei m einen Zahlenwert von 0,001 bis 20 besitzt, bevorzugt 0,1 bis 20, besonders bevorzugt 0,5 bis 20, ganz besonders bevorzugt für 0,5 bis 5.

Bei dem Di- und/oder Poly(hydrogen)fluorid ([F$^-$ x HF)$_m$]) kann es sich insbesondere um ein quaternäres Ammoniumfluorid, Ammoniumdifluorid, Ammoniumtrifluorid, ein höheres Ammoniumpolyfluorid, ein Phosphoniumfluorid, ein Phosphoniumdifluorid, ein Phosphoniumtrifluorid und/oder um ein höheres Phosphoniumpolyfluorid handeln, vorzugsweise um solche, wie sie durch Abmischen von quaternären Ammonium- sowie Phosphonium-fluoriden oder -hydroxiden mit entsprechenden Mengen, optional in Alkoholen oder Wasser vorgelöstem Fluorwasserstoff bereitet werden können.

Als Lösungsmittel für den/die Katalysatoren kommen alle Verbindungen in Frage, die nicht mit dem Katalysator reagieren und ihn in ausreichendem Maße zu lösen vermögen. Dies sind z.B. für die o.g. Tetraorganyl-ammoniumsalze und -phosphoniumsalze aliphatische oder aromatische Kohlenwasserstoffe, Alkohole, Ester sowie Ether. Bevorzugt werden Alkohole verwendet.

Der Katalysatorbedarf im erfindungsgemäßen Verfahren unterscheidet sich dabei nicht signifikant von dem in der Bulk-Modifizierung des Standes der Technik beobachteten. Der Katalysator/die Katalysatormischung kann beispielsweise in einem Anteil von 1 mol-ppm bis 1 mol-%, bevorzugt 5 mol-ppm bis 0,1 mol-%, jeweils bezogen auf die Menge an monomerem Di- und/oder Triisocyanat eingesetzt werden.

Das erfindungsgemäße Verfahren kann beispielsweise im Temperaturbereich von 0 °C bis + 250 °C durchgeführt werden, bevorzugt 20 bis 180 °C, besonders bevorzugt 40 bis 150 °C.

In weiterer Ausgestaltung des erfindungsgemäßen Verfahrens kann die Oligomerisierung abgebrochen werden, nachdem 5 bis 80 Gew.-% des eingesetzten monomeren Di- und/oder Triisocyanates umgesetzt sind, bevorzugt 10 bis 60 Gew.-%. Die Oligomerisierung kann beispielsweise abgebrochen werden, indem der Katalysator desaktiviert wird. Zur Katalysatordeaktivierung bieten sich prinzipiell eine ganze Reihe vorbeschriebener Methoden des Standes der Technik an, wie z.B. die Zugabe (unter- oder über-) stöchiometrischer Mengen an Säuren oder Säurederivaten (z.B. Benzoylchlorid, saure Ester Phosphor oder Schwefel enthaltender Säuren, diese Säuren selbst etc., nicht jedoch HF), adsorptive Bindung des Katalysators und anschließende Abtrennung durch Filtration oder Kombinationen hiervon.

Im Anschluss an die Katalysatordesaktivierung kann das nicht umgesetzte Monomer sowie gegebenenfalls mitverwendetes Lösungsmittel mit Hilfe aller bekannten Separationstechniken wie z.B. Destillation, gegebenenfalls in der speziellen Ausführungsform der Dünnschichtdestillation Extraktion oder Kristallisation/Filtration abgetrennt werden.

Selbstverständlich können auch Kombinationen zweier oder mehrerer dieser Techniken angewendet werden.

Soll das erfindungsgemäß hergestellte Polyisocyanat noch freies, nicht umgesetztes Monomer enthalten, wie es z.B. für die Weiterverarbeitung zu NCO-blockierten Produkten von Interesse ist, so kann nach Katalysatordeaktivierung auf die Monomerenabtrennung verzichtet werden.

Bevorzugt wird das nicht umgesetzte Monomer abgetrennt, insbesondere destillativ. In weiter bevorzugter Weise weisen die erfindungsgemäßen Produkte nach der Abtrennung einen Restmonomergehalt < 0,5 Gew.-% auf, bevorzugt < 0,25 Gew.- %, besonders bevorzugt < 0,1 Gew.- %.

Verglichen mit der Katalyse z.B. durch quaternäre Phosphoniumsalze ohne Verwendung von Additiven (Bulk-Modifizierung, s. Vergleichsbeispiel), beobachtet man im erfindungsgemäßen Verfahren bei sonst gleichen Reaktionsbedingungen eine deutlich verbesserte Katalysatorstabilität was sich in deutlich niedrigeren Phosphorgehalten des Recyclates äußert.

Nach einer weiter bevorzugten, kontinuierlich arbeitenden Ausführungsform des erfindungsgemäßen Verfahrens kann die Oligomerisierung in einem Rohrreaktor vorgenommen werden. Dies ist deshalb vorteilhaft, da sich hierbei eine signifikant geringere Neigung der erfindungsgemäßen Katalysatoren spontan Gelteilchen im Produkt zu bilden im Vergleich zu den bekannten Katalysatoren des Standes der Technik ergibt, selbst bei Applikation in hochkonzentrierter Lösung bzw. als reiner Wirkstoff.

Die vorliegende Erfindung betrifft ferner ein Reaktionssystem zur Herstellung von Iminooxadiazindiongruppen enthaltenden Polyisocyanaten, umfassend mindestens ein monomeres Di- und/oder Triisocyanat, sowie

a) mindestens einem Katalysator,
b) mindestens ein Additiv (A) mit einer relativen Permittivität bei 18 °C bis 30 °C von wenigstens 4,0, insbesondere von wenigstens 8,0, bevorzugt von wenigstens 20,0, besonders bevorzugt von wenigstens 30,0 oder gar wenigstens 35,0,
c) gegebenenfalls weitere, von (A) verschiedene Additive,

wobei 1 bis 50 Gew.-% an Additiv (A), bezogen auf die Masse des monomeren Di- und/oder Triisocyanats, eingesetzt werden.

Ein weiterer Gegenstand der vorliegenden Erfindung betrifft die Verwendung von Verbindungen mit einer relativen Permittivität bei 18 °C bis 30 °C von wenigstens 4,0, insbesondere von wenigstens 8,0, bevorzugt von wenigstens 20,0, besonders bevorzugt von wenigstens 30,0 oder gar wenigstens 35,0 als Additiv (A) zur Herstellung von Iminooxadiazindiongruppen enthaltenden Polyisocyanaten durch katalysierte Modifizierung monomerer Di- und/oder Triisocyanate, wobei 1 bis 50 Gew.-% an Additiv (A), bezogen auf die Masse der monomeren Di- und/oder Triisocyanate, eingesetzt werden.

Die nach dem erfindungsgemäßen Verfahren erhältlichen Produkte bzw. Produktgemische stellen mithin vielseitig verwendbare Ausgangsmaterialien zur Herstellung von, ggf. geschäumte(n), Kunststoffe(n) sowie Lacken, Beschichtungsmitteln, Klebstoffen und Zuschlagstoffen dar. Insbesondere zur Herstellung von, ggf. wasserdipergierbaren Ein- und Zweikomponenten-Polyurethanlacken eignen sie sich, ggf. in NCO-blockierter Form, aufgrund ihrer im Vergleich zu (überwiegend) Isocyanurat-Polyisocyanat basierenden Produkten verringerten Lösungs- sowie Schmelzviskosität bei ansonsten gleich hohem bzw. verbessertem Eigenschaftsprofil. So sind die erfindungsgemäßen Verfahrensprodukte auf HDI-Basis auch in hoher Verdünnung in Lacklösungsmitteln stabiler gegen das Auftreten von Ausflockungen bzw. Trübungen, als entsprechende Produkte auf Isocyanuratbasis.

Sie können rein oder in Verbindung mit anderen Isocyanatderivaten des Standes der Technik, wie z.B. Uretdion-, Biuret-, Allophanat-, Isocyanurat- und/oder Urethan-Gruppen enthaltenden Polyisocyanaten, deren freie NCO-Gruppen ggf. mit Blockierungsmitteln desaktiviert wurden, eingesetzt werden.

**Beispiele**

Die vorliegende Erfindung wird im Folgenden anhand von Beispielen näher erläutert.

Alle Mengenangaben beziehen sich, soweit nicht anders vermerkt, auf die Masse.

Die Ermittlung des NCO-Gehaltes der in den Beispielen und Vergleichsbeispielen beschriebenen Harze erfolgte durch Titration gemäß DIN 53 185.

Der Phosphorgehalt aller Proben wurde durch Röntgenfluoreszenzanalyse (RFA) bestimmt.

Mol-% Angaben wurden NMR-spektroskopisch ermittelt und beziehen sich immer, so nicht anders ausgewiesen, auf die Summe der NCO-Folgeprodukte. Die Messungen erfolgten auf den Geräten DPX 400 bzw. DRX 700 der Fa. Brucker an ca. 5 %igen ($^1$H-NMR) bzw. ca. 50 %igen ($^{13}$C-NMR) Proben in trockenem $C_6D_6$ bei einer Frequenz von 400 bzw. 700 MHz ($^1$H-NMR) oder 100 bzw. 176 MHz ($^{13}$C-NMR). Als Referenz für die ppm-Skala wurden geringe Mengen von Tetramethylsilan im Lösungsmittel mit 0 ppm $^1$H-NMR-chem. Verschiebung heran gezogen. Alternativ wurde auf das Signal des im Lösungsmittel enthaltenen $C_6D_5H$ referenziert: 7,15 ppm $^1$H-NMR-chem. Verschiebung,

128,02 ppm $^{13}$C-NMR-chem. Verschiebung.. Daten für die chemische Verschiebung der in Frage kommenden Verbindungen wurden der Literatur entnommen (vgl. D. Wendisch, H. Reiff und D. Dieterich, Die Angewandte Makromolekulare Chemie 141, 1986, 173-183 und darin zit. Lit sowie EP-A 896 009.

Die dynamischen Viskositäten wurden bei 23 °C mit dem Viskosimeter VT 550 der Fa. Haake bestimmt. Durch Messungen bei unterschiedlichen Schergeschwindigkeiten wurde sichergestellt, daß das Fließverhalten der beschriebenen erfindungsgemäßen Polyisocyanatmischungen wie auch das der Vergleichsprodukte dem idealer Newtonscher Flüssigkeiten entspricht. Die Angabe der Schergeschwindigkeit kann deshalb entfallen.

Die Bestimmung der Restmonomergehalte erfolgte gaschromatographisch.

Alle Reaktionen wurden, wenn nicht anders angegeben, unter einer Stickstoffatmosphäre durchgeführt.

Die verwendeten Diisocyanate sind Produkte der Bayer MaterialScience AG, D-51368 Leverkusen, alle anderen kommerziell zugänglichen Chemikalien wurden von der Fa. Aldrich, D-82018 Taufkirchen, bezogen.

**Beispiel 1 Vergleichsbeispiel**

In einem doppelwandigen, durch einen externen Kreislauf auf die jeweils gewünschte Starttemperatur temperierten Planschliffgefäß mit Rührer, an eine Inertgasanlage (Stickstoff/Vakuum) angeschlossenem Rückflusskühler und Thermometer wurden 1000 g HDI vorgelegt und durch einstündiges Rühren im Vakuum (0,1 mbar) von gelösten Gasen befreit. Nach Belüften mit Stickstoff wurde die in Tabelle **1** angegebene Katalysatormenge (bezogen auf die Masse an eingesetztem HDI, als 70%ige Lösung in Isopropanol) portionsweise so dosiert, dass die in Tabelle 1 angegebene Maximaltemperatur nicht überschritten wurde. Nachdem ca. 1 mol NCO Gruppen umgesetzt waren, wurde der Katalysator durch Zugabe einer zum Katalysator äquivalenten Menge an p-Toluolsulfonsäure (als 40%ige Lösung in Isopropanol) deaktiviert, weitere 30 min bei Reaktionstemperatur nachgerührt und anschließend aufgearbeitet. Die Aufarbeitung erfolgte durch Vakuumdestillation in einem Dünnschichtverdampfer, Typ Kurzwegverdampfer (KWV), mit vorgeschaltetem Vorverdampfer (VV) (Destillationsdaten: Druck: 0,08 +/- 0,04 mbar, VV-Temperatur: 120°C, KWV-Temp.: 140°C), wobei nicht umgesetztes Monomer als Destillat und das monomerenarme Polyisocyanatharz als Sumpfprodukt separiert wurden (Startdurchlauf: Beispiel 1-A).

Das Polyisocyanatharz wurde separiert und das Destillat in einer zweiten Planschliff-Rührapparatur, die identisch zur ersten aufgebaut ist, gesammelt und mit frisch entgastem HDI auf die Ausgangsmenge (1000 g) aufgefüllt. Anschließend wurde erneut katalysiert und verfahren wie eingangs beschrieben. Diese Verfahrensweise wurde mehrmals wiederholt. Die Ergebnisse sind Tabelle 1 zu entnehmen. Aus der Analyse der Phosphorgehalte der erhaltenen Polyisocyanatharze und des am Ende der Versuchsserie verbleibenden Recyclatmonomers wurde die Phosphorbilanz ermittelt. Sie beläuft sich bei summarisch 90 % Wiederfindung auf 83 % des gefundenen Phosphors in den Harzen und 17 % im letzten Destillat. Die gemittelten Daten der in den Versuchen 1-B bis 1-F erhaltenen Polyisocyanatharze lauten wie folgt:

| | |
|---|---|
| Harzausbeute (bezogen auf eingesetztes HDI): | 17,6% |
| NCO-Gehalt: | 23,4 % |
| Viskosität: | 700 mPas/23°C |
| Iminooxadiazindione: | 51 mol-%* |
| Isocyanurate: | 43 mol-%* |
| Uretdione: | 6 mol-%* |

* = bezogen auf die Summe der in der Modifizierungsreaktion gebildeten NCO-Folgeprodukte

Tabelle 1

| Beispiel-Nr. | | Katalysator- | | | Reaktionstemperatur [°C] | |
|---|---|---|---|---|---|---|
| | | kation | anion | menge [ppm] | Start | Max. |
| 1- | A | n-Bu$_4$P$^+$ | [HF$_2$]$^-$ | 310 | 60 | 64 |
| 1- | B | n-Bu$_4$P$^+$ | [HF$_2$]$^-$ | 282 | 60 | 64 |
| 1- | C | n-Bu$_4$P$^+$ | [HF$_2$]$^-$ | 316 | 60 | 67 |
| 1- | D | n-Bu$_4$P$^+$ | [HF$_2$]$^-$ | 304 | 60 | 66 |
| 1- | E | n-Bu$_4$P$^+$ | [HF$_2$]$^-$ | 284 | 60 | 67 |

(fortgesetzt)

| Beispiel-Nr. | | Katalysator- | | | Reaktionstemperatur [°C] | |
|---|---|---|---|---|---|---|
| | | kation | anion | menge [ppm] | Start | Max. |
| 1- | F | n-Bu$_4$P$^+$ | [HF$_2$]$^-$ | 289 | 60 | 65 |

**Beispiel 2 erfindungsgemäß**

Additiv: Acetonitril (relative Permittivität bei 20 °C/ 50 Hz: 37,5)

Es wurde Verfahren wie in Beispiel 1 beschrieben, mit dem Unterschied, dass dem entgasten HDI 20 % Acetonitril zugesetzt wurden und das das Acetonitril nach der jeweiligen Reaktion und vor der Vakuumdestillation durch Passage durch den auf 120°C (VV) und 140°C (KWV) geheizten Destillationsapparat bei Normaldruck abgetrennt und in den nächsten Ansatz dosiert wurde. Anschließend erfolgte die destillative Auftrennung von Recyclatmonomer und Polyisocyanatharz wie in Bsp. 1 beschrieben.

Aus der Analyse der Phosphorgehalte der erhaltenen Polyisocyanatharze und des am Ende der Versuchsserie verbleibenden Recyclatmonomers (incl. Additiv) wurde die Phosphorbilanz ermittelt. Sie beläuft sich bei summarisch 90 % Wiederfindung auf 97 % des gefundenen Phosphors in den Harzen und 3 % im letzten Destillat. Die gemittelten Daten der in den Versuchen 2-B bis 2-F erhaltenen Polyisocyanatharze lauten wie folgt:

| | |
|---|---|
| Harzausbeute (bezogen auf eingesetztes HDI): | 17,3 % |
| NCO-Gehalt: | 23,5 % |
| Viskosität: | 609 mPas/23°C |
| Iminooxadiazindione: | 48 mol-%* |
| Isocyanurate: | 43 mol-%* |
| Uretdione: | 9 mol-%* |

\* = bezogen auf die Summe der in der Modifizierungsreaktion gebildeten NCO-Folgeprodukte

Tabelle 2

| Beispiel-Nr. | | Katalysator- | | | Reaktionstemperatur [°C] | |
|---|---|---|---|---|---|---|
| | | kation | anion | menge [ppm] | Start | Max. |
| 2- | A | n-Bu$_4$P$^+$ | [HF$_2$]$^-$ | 477 | 60 | 62 |
| 2- | B | n-Bu$_4$P$^+$ | [HF$_2$]$^-$ | 455 | 60 | 62 |
| 2- | C | n-Bu$_4$P$^+$ | [HF$_2$]$^-$ | 471 | 60 | 61 |
| 2- | D | n-Bu$_4$P$^+$ | [HF$_2$]$^-$ | 455 | 60 | 62 |
| 2- | E | n-Bu$_4$P$^+$ | [HF$_2$]$^-$ | 474 | 60 | 62 |
| 2- | F | n-Bu$_4$P$^+$ | [HF$_2$]$^-$ | 478 | 60 | 61 |

**Beispiel 3 erfindungsgemäß**

Additiv: Adiponitril (relative Permittivität bei 18 °C/ 50 Hz: 30,0)

Es wurde Verfahren wie in Beispiel 1 beschrieben, mit dem Unterschied, dass dem entgasten HDI 5 % Adiponitril zugesetzt wurden. Da Adiponitril eine dem HDI vergleichbare Flüchtigkeit aufweist, wurde bei der Aufarbeitung verfahren wie in Bsp. **1** beschrieben.

Aus der Analyse der Phosphorgehalte der erhaltenen Polyisocyanatharze und des am Ende der Versuchsserie verbleibenden Recyclatmonomers (incl. Additiv) wurde die Phosphorbilanz ermittelt. Sie beläuft sich bei summarisch 91 % Wiederfindung auf 93 % des gefundenen Phosphors in den Harzen und 7 % im letzten Destillat. Die gemittelten Daten der in den Versuchen 3-B bis 3-F erhaltenen Polyisocyanatharze lauten wie folgt:

| Harzausbeute (bezogen auf eingesetztes HDI): | 20,2 % |
| NCO-Gehalt: | 23,6 % |
| Viskosität: | 740 mPas/23°C |
| Iminooxadiazindione: | 52 mol-%* |
| Isocyanurate: | 43 mol-%* |
| Uretdione: | 5 mol-%* |

\* = bezogen auf die Summe der in der Modifizierungsreaktion gebildeten NCO-Folgeprodukte

Tabelle 3

| Beispiel-Nr. | | Katalysator- | | | Reaktionstemperatur [°C] | |
|---|---|---|---|---|---|---|
| | | Kation | anion | menge [ppm] | Start | Max. |
| 3- | A | n-Bu$_4$P$^+$ | [HF$_2$]$^-$ | 331 | 60 | 60,8 |
| 3- | B | n-Bu$_4$P$^+$ | [HF$_2$]$^-$ | 319 | 60 | 64,2 |
| 3- | C | n-Bu$_4$P$^+$ | [HF$_2$]$^-$ | 337 | 60 | 64,5 |
| 3- | D | n-Bu$_4$P$^+$ | [HF$_2$]$^-$ | 282 | 60 | 63,8 |
| 3- | E | n-Bu$_4$P$^+$ | [HF$_2$]$^-$ | 324 | 60 | 64,8 |
| 3- | F | n-Bu$_4$P$^+$ | [HF$_2$]$^-$ | 344 | 60 | 64,8 |

**Beispiel 4 erfindungsgemäß**

Additiv: Propylencarbonat (relative Permittivität bei 25 °C/ 50 Hz: 65,1)

Es wurde Verfahren wie in Beispiel 1 beschrieben, mit dem Unterschied, dass dem entgasten HDI 5 % Propylencarbonat zugesetzt wurden. Da Propylencarbonat eine dem HDI vergleichbare Flüchtigkeit aufweist, wurde bei der Aufarbeitung verfahren wie in Bsp. 1 beschrieben.

Aus der Analyse der Phosphorgehalte der erhaltenen Polyisocyanatharze und des am Ende der Versuchsserie verbleibenden Recyclatmonomers (incl. Additiv) wurde die Phosphorbilanz ermittelt. Sie beläuft sich bei summarisch 90 % Wiederfindung auf 91 % des gefundenen Phosphors in den Harzen und 9 % im letzten Destillat. Die gemittelten Daten der in den Versuchen 4-B bis 4-F erhaltenen Polyisocyanatharze lauten wie folgt:

| Harzausbeute (bezogen auf eingesetztes HDI): | 21,6 % |
| NCO-Gehalt: | 23,3 % |
| Viskosität: | 780 mPas/23°C |
| Iminooxadiazindione: | 52 mol-%* |
| Isocyanurate: | 42 mol-%* |
| Uretdione: | 6 mol-%* |

\* = bezogen auf die Summe der in der Modifizierungsreaktion gebildeten NCO-Folgeprodukte

Tabelle 4

| Beispiel-Nr. | | Katalysator- | | | Reaktionstemperatur [°C] | |
|---|---|---|---|---|---|---|
| | | kation | anion | menge [ppm] | Start | Max. |
| 4- | A | n-Bu$_4$P$^+$ | [HF$_2$]$^-$ | 351 | 60 | 65 |
| 4- | B | n-Bu$_4$P$^+$ | [HF$_2$]$^-$ | 300 | 60 | 64 |
| 4- | C | n-Bu$_4$P$^+$ | [HF$_2$]$^-$ | 309 | 60 | 65 |

(fortgesetzt)

| Beispiel-Nr. | | Katalysator- | | | Reaktionstemperatur [°C] | |
|---|---|---|---|---|---|---|
| | | kation | anion | menge [ppm] | Start | Max. |
| 4- | D | n-Bu$_4$P$^+$ | [HF$_2$]$^-$ | 300 | 60 | 65 |
| 4- | E | n-Bu$_4$P$^+$ | [HF$_2$]$^-$ | 311 | 60 | 64 |
| 4- | F | n-Bu$_4$P$^+$ | [HF$_2$]$^-$ | 323 | 60 | 65 |

**Beispiel 5 erfindungsgemäß**

Additiv: gamma-Valerolacton (relative Permittivität bei 20 °C/ 50 Hz: 36,9)

Es wurde Verfahren wie in Beispiel 1 beschrieben, mit dem Unterschied, dass dem entgasten HDI 5 % gamma-Valerolacton zugesetzt wurden. Da gamma-Valerolacton eine dem HDI vergleichbare Flüchtigkeit aufweist, wurde bei der Aufarbeitung verfahren wie in Bsp. 1 beschrieben.

Aus der Analyse der Phosphorgehalte der erhaltenen Polyisocyanatharze und des am Ende der Versuchsserie verbleibenden Recyclatmonomers (incl. Additiv) wurde die Phosphorbilanz ermittelt. Sie beläuft sich bei summarisch 98 % Wiederfindung auf 90 % des gefundenen Phosphors in den Harzen und 10 % im letzten Destillat. Die gemittelten Daten der in den Versuchen 5-B bis 5-F erhaltenen Polyisocyanatharze lauten wie folgt:

| | |
|---|---|
| Harzausbeute (bezogen auf eingesetztes HDI): | 20,9 % |
| NCO-Gehalt: | 23,2 % |
| Viskosität: | 745 mPas/23°C |
| Iminooxadiazindione: | 49 mol-%* |
| Isocyanurate: | 44 mol-%* |
| Uretdione: | 7 mol-%* |

\* = bezogen auf die Summe der in der Modifizierungsreaktion gebildeten NCO-Folgeprodukte

Tabelle 5

| Beispiel-Nr. | | Katalysator- | | | Reaktionstemperatur [°C] | |
|---|---|---|---|---|---|---|
| | | kation | anion | menge [ppm] | Start | Max. |
| 5- | A | n-Bu$_4$P$^+$ | [HF$_2$]$^-$ | 389 | 60 | 61 |
| 5- | B | n-Bu$_4$P$^+$ | [HF$_2$]$^-$ | 329 | 60 | 65 |
| 5- | C | n-Bu$_4$P$^+$ | [HF$_2$]$^-$ | 301 | 60 | 64 |
| 5- | D | n-Bu$_4$P$^+$ | [HF$_2$]$^-$ | 314 | 60 | 65 |
| 5- | E | n-Bu$_4$P$^+$ | [HF$_2$]$^-$ | 298 | 60 | 65 |
| 5- | F | n-Bu$_4$P$^+$ | [HF$_2$]$^-$ | 298 | 60 | 65 |

**Beispiel 6 Vergleichsbeispiel**

Additiv: Isoeicosan (relative Permittivität bei 25 °C/ 50 Hz: 2,1)

Es wurde Verfahren wie eingangs beschrieben, mit dem Unterschied, dass dem entgasten HDI 20 % Isoeicosan (Isomerengemisch) zugesetzt wurden. Da Isoeicosan eine dem HDI vergleichbare Flüchtigkeit aufweist, wurde bei der Aufarbeitung verfahren wie in Bsp. 1 beschrieben.

Aus der Analyse der Phosphorgehalte der erhaltenen Polyisocyanatharze und des am Ende der Versuchsserie verbleibenden Recyclatmonomers (incl. Additiv) wurde die Phosphorbilanz ermittelt. Sie beläuft sich bei summarisch 81 % Wiederfindung auf 82 % des gefundenen Phosphors in den Harzen und 18 % im letzten Destillat. Die gemittelten Daten der in den Versuchen 6-B bis 6-F erhaltenen Polyisocyanatharze lauten wie folgt:

| Harzausbeute (bezogen auf eingesetztes HDI): | 17,1 % |
| NCO-Gehalt: | 23,6 % |
| Viskosität: | 700 mPas/23°C |
| Iminooxadiazindione: | 57,7 mol-%* |
| Isocyanurate: | 38,5 mol-%* |
| Uretdione: | 3,8 mol-%* |

\* = bezogen auf die Summe der in der Modifizierungsreaktion gebildeten NCO-Folgeprodukte

Tabelle 6

| Beispiel-Nr. | | Katalysator- | | | Reaktionstemperatur [°C] | |
|---|---|---|---|---|---|---|
| | | kation | anion | menge [ppm] | Start | Max. |
| 6- | A | $n\text{-}Bu_4P^+$ | $[HF_2]^-$ | 471 | 60 | 61 |
| 6- | B | $n\text{-}Bu_4P^+$ | $[HF_2]^-$ | 423 | 60 | 60 |
| 6- | C | $n\text{-}Bu_4P^+$ | $[HF_2]^-$ | 423 | 60 | 61 |
| 6- | D | $n\text{-}Bu_4P^+$ | $[HF_2]^-$ | 439 | 60 | 63 |
| 6- | E | $n\text{-}Bu_4P^+$ | $[HF_2]^-$ | 423 | 60 | 63 |
| 6- | F | $n\text{-}Bu_4P^+$ | $[HF_2]^-$ | 423 | 60 | 63 |

Wie man sofort erkennt, hat der Zusatz des unpolaren Additivs Isoeicosan keinerlei positiven Einfluß auf die Zersetzungstendenz des Katalysators und liefert gegenüber der Reaktion in reinem HDI (Vergleichsbeispiel 1) identische Resultate.

**Patentansprüche**

1.  Verfahren zur Herstellung von Iminooxadiazindiongruppen enthaltenden Polyisocyanaten, bei dem mindestens ein monomeres Di- und/oder Triisocyanat, in Gegenwart von

    a) mindestens einem Katalysator,
    b) mindestens einem Additiv (A) mit einer relativen Permittivität bei 18 °C bis 30°C von wenigstens 4,0, gemessen wie in der Beschreibung angegeben,
    c) gegebenenfalls weiteren, von A verschiedenen Additiven oligomerisiert wird, wobei 1 bis 50 Gew.-% an Additiv (A), bezogen auf die Masse des monomeren Di- und/oder Triisocyanats, eingesetzt werden.

2.  Verfahren nach Anspruch 1, **dadurch gekennzeichnet, dass** dem monomeren Di- und/oder Triisocyanat das Additiv (A) vor dem Inkontaktbringen mit dem Katalysator zugesetzt wird.

3.  Verfahren nach Anspruch 1 oder 2, **dadurch gekennzeichnet, dass** als Additiv (A) Nitrile, Carbonate und/oder cyclische Lactone eingesetzt werden, insbesondere ausgewählt aus der Gruppe umfassend Acetonitril, Adiponitril, Ethlyencarbonat Propylencarbonat und gamma-Valerolacton.

4.  Verfahren nach einem der vorstehenden Ansprüche, **dadurch gekennzeichnet, dass** 2 bis 30 Gew.-%, besonders bevorzugt 2 bis 20 Gew.-% an Additiv (A), bezogen auf die Masse des monomeren Di- und/oder Triisocyanats eingesetzt werden .

5.  Verfahren nach einem der vorstehenden Ansprüche, **dadurch gekennzeichnet, dass** das Additiv (A) eine relative Permittivität bei 18 °C bis 30 °C von wenigstens 8,0 aufweist, bevorzugt von wenigstens 20,0, besonders bevorzugt von wenigstens 30,0 oder gar wenigstens 35,0.

6.  Verfahren nach einem der vorstehenden Ansprüche, **dadurch gekennzeichnet, dass** als monomeres Di- und/oder Triisocyanat ein aliphatisches Diisocyanat eingesetzt wird, insbesondere Hexamethylendiisocyanat (HDI), 2-Methylpentan-1,5-diisocyanat, 2,4,4-Trimethyl-1,6-hexandiisocyanat, 2,2,4-Trimethyl-1,6-hexandiisocyanat und/oder 4-Isocyanatomethyl-1,8-octandiisocyanat, bevorzugt HDI.

7. Verfahren nach einem der vorstehenden Ansprüche, **dadurch gekennzeichnet, dass** als Katalysator ein Tetraorganyl-ammoniumsalz und/oder -phosphoniumsalz eingesetzt wird, wobei die Anionen des Tetraorganyl-ammoniumsalzes und/oder - phosphoniumsalzes insbesondere ausgewählt sind aus der Gruppe: $RfCR_1R_2COO^-$, wobei Rf für einen geradkettigen oder verzweigten Perfluoralkylrest und $R_1$ und $R_2$ unabhängig voneinander für H, geradkettige oder verzweigte Organylreste stehen, Fluorid ($F^-$), Di- und/oder Poly(hydrogen)fluoride ($[F^- \times HF]_m$), wobei m einen Zahlenwert von 0,001 bis 20 besitzt, bevorzugt 0,1 bis 20, besonders bevorzugt 0,5 bis 20, ganz besonders bevorzugt für 0,5 bis 5.

8. Verfahren nach Anspruch 7, **dadurch gekennzeichnet, dass** es sich bei dem Di- und/oder Poly(hydrogen)fluorid ($[F^- \times HF]_m$) um ein quaternäres Ammoniumfluorid, Ammoniumdifluorid, Ammoniumtrifluorid, ein höheres Ammoniumpolyfluorid, ein Phosphoniumfluorid, ein Phosphoniumdifluorid, ein Phosphoniumtrifluorid und/oder um ein höheres Phosphoniumpolyfluorid handelt, vorzugsweise um solche, wie sie durch Abmischen von quaternären Ammonium- sowie Phosphonium-fluoriden oder - hydroxiden mit entsprechenden Mengen, optional in Alkoholen oder Wasser vorgelöstem Fluorwasserstoff bereitet werden können.

9. Verfahren nach einem der vorstehenden Ansprüche, **dadurch gekennzeichnet, dass** der Katalysator/die Katalysatormischung in einem Anteil von 1 mol-ppm bis 1 mol-%, bevorzugt 5 mol-ppm bis 0,1 mol-%, jeweils bezogen auf die Menge an monomerem Di- und/oder Triisocyanat eingesetzt wird.

10. Verfahren nach einem der vorstehenden Ansprüche, **dadurch gekennzeichnet, dass** das Verfahren im Temperaturbereich von 0 °C bis + 250 °C durchgeführt wird, bevorzugt 20 bis 180 °C, besonders bevorzugt 40 bis 150 °C.

11. Verfahren nach einem der vorstehenden Ansprüche, **dadurch gekennzeichnet, dass** die Oligomerisierung abgebrochen wird, nachdem 5 bis 80 Gew.-% des eingesetzten monomeren Di- und/oder Triisocyanates umgesetzt sind, bevorzugt 10 bis 60 Gew.-%.

12. Verfahren nach Anspruch 11, **dadurch gekennzeichnet, dass** die Oligomerisierung abgebrochen wird, indem der Katalysator desaktiviert wird, insbesondere durch Zugabe einer Säure oder eines Säurederivates wie Benzoylchlorid, eines sauren Esters Phosphor oder Schwefel enthaltender Säuren, diese Säuren selbst, adsorptive Bindung des Katalysators und anschließende Abtrennung durch Filtration oder Kombinationen hiervon.

13. Verfahren nach Anspruch 11 oder 12, **dadurch gekennzeichnet, dass** nicht umgesetztes Monomer aus der Reaktionsmischung abgetrennt wird.

14. Reaktionssystem zur Herstellung von Iminooxadiazindiongruppen enthaltenden Polyisocyanaten, umfassend mindestens ein monomeres Di- und/oder Triisocyanat, sowie

   a) mindestens einem Katalysator,
   b) mindestens ein Additiv (A) mit einer relativen Permittivität v bei 18 °C bis 30 °C on wenigstens 4,0, gemessen wie in der Beschreibung angegeben,
   c) gegebenenfalls weitere, von A verschiedene Additive,

wobei 1 bis 50 Gew.-% an Additiv (A), bezogen auf die Masse des monomeren Di- und/oder Triisocyanats, eingesetzt werden.

15. Verwendung von Verbindungen mit einer relativen Permittivität bei 18 °C bis 30 °C von wenigstens 4,0, gemessen wie in der Beschreibung angegeben, als Additiv (A) zur Herstellung von Iminooxadiazindiongruppen enthaltenden Polyisocyanaten durch katalysierte Modifizierung monomerer Di- und/oder Triisocyanate, wobei 1 bis 50 Gew.-% an Additiv (A), bezogen auf die Masse der monomeren Di- und/oder Triisocyanate, eingesetzt werden.

**Claims**

1. Method for producing polyisocyanates comprising iminooxadiazinedione groups, wherein at least one monomeric di- and/or tri-isocyanate is oligomerised in the presence of

a) at least one catalyst,
b) at least one additive (A) having a relative permittivity at 18°C to 30°C of at least 4.0,
c) optionally further additives other than A measured as stated in the description,

wherein there are used from 1 to 50 wt. % of additive (A), based on the mass of the monomeric di- and/or tri-isocyanate.

2. Method according to Claim 1, **characterised in that** the additive (A) is added to the monomeric di- and/or tri-isocyanate before it is brought into contact with the catalyst.

3. Method according to Claim 1 or 2, **characterised in that** there are used as the additive (A) nitriles, carbonates and/or cyclic lactones, in particular selected from the group comprising acetonitrile, adiponitrile, ethylene carbonate, propylene carbonate and gamma-valerolactone.

4. Method according to any one of the preceding claims, **characterised in that** there are used from 2 to 30 wt.%, particularly preferably from 2 to 20 wt.%, of additive (A), based on the mass of the monomeric di- and/or tri-isocyanate.

5. Method according to any one of the preceding claims, **characterised in that** the additive (A) has a relative permittivity at 18°C to 30°C of at least 8.0, preferably of at least 20.0, particularly preferably of at least 30.0 or even at least 35.0.

6. Method according to any one of the preceding claims, **characterised in that** there is used as the monomeric di- and/or tri-isocyanate an aliphatic diisocyanate, in particular hexamethylene diisocyanate (HDI), 2-methylpentane 1,5-diisocyanate, 2,4,4-trimethyl-1,6-hexane diisocyanate, 2,2,4-trimethyl-1,6-hexane diisocyanate and/or 4-isocyanatomethyl-1,8-octane diisocyanate, preferably HDI.

7. Method according to any one of the preceding claims, **characterised in that** there is used as the catalyst a tetraorganyl-ammonium salt and/or - phosphonium salt, wherein the anions of the tetraorganyl-ammonium salt and/or -phosphonium salt are selected in particular from the group: $RfCR_1R_2COO^-$, wherein Rf represents a straight-chained or branched perfluoroalkyl radical and $R_1$ and $R_2$ independently of one another represent H, straight-chained or branched organyl radicals, fluoride ($F^-$), di- and/or poly-(hydrogen) fluorides ($[F^- \times HF_m]$), wherein m has a numerical value of from 0.001 to 20, preferably from 0.1 to 20, particularly preferably from 0.5 to 20, most particularly preferably from 0.5 to 5.

8. Method according to Claim 7, **characterised in that** the di- and/or poly-(hydrogen) fluoride ($[F^- \times HF_m]$) is a quaternary ammonium fluoride, ammonium difluoride, ammonium trifluoride, a higher ammonium polyfluoride, a phosphonium fluoride, a phosphonium difluoride, a phosphonium trifluoride and/or a higher phosphonium polyfluoride, preferably those which can be prepared by mixing quaternary ammonium and phosphonium fluorides or hydroxides with corresponding amounts of hydrogen fluoride, optionally pre-dissolved in alcohols or water.

9. Method according to any one of the preceding claims, **characterised in that** the catalyst/catalyst mixture is used in an amount of from 1 mol-ppm to 1 mol-%, preferably from 5 mol-ppm to 0.1 mol-%, in each case based on the amount of monomeric di- and/or tri-isocyanate.

10. Method according to any one of the preceding claims, **characterised in that** the method is carried out in the temperature range of from 0°C to +250°C, preferably from 20 to 180°C, particularly preferably from 40 to 150°C.

11. Method according to any one of the preceding claims, **characterised in that** the oligomerisation is terminated when from 5 to 80 wt.%, preferably from 10 to 60 wt.%, of the monomeric di- and/or tri-isocyanate used has been converted.

12. Method according to Claim 11, **characterised in that** the oligomerisation is terminated by deactivation of the catalyst, in particular by addition of an acid or of an acid derivative such as benzoyl chloride, an acid ester of acids containing phosphorus or sulfur, those acids themselves, adsorptive binding of the catalyst and subsequent separation by filtration, or combinations thereof.

13. Method according to Claim 11 or 12, **characterised in that** unconverted monomer is separated from the reaction mixture.

**14.** Reaction system for producing polyisocyanates comprising iminooxadiazinedione groups, which reaction system comprises at least one monomeric di- and/or tri-isocyanate as well as

a) at least one catalyst,
b) at least one additive (A) having a relative permittivity v at 18°C to 30°C of at least 4.0, measured as stated in the description,
c) optionally further additives other than A, wherein there are used from 1 to 50 wt. % of additive (A), based on the mass of the monomeric di- and/or tri-isocyanate.

**15.** Use of compounds having a relative permittivity at 18°C to 30°C of at least 4.0, measured as stated in the description, as an additive (A) for producing polyisocyanates comprising iminooxadiazinedione groups by catalysed modification of monomeric di- and/or triisocyanates, wherein there are used from 1 to 50 wt. % of additive (A), based on the mass of the monomeric di- and/or tri-isocyanate.

**Revendications**

**1.** Procédé pour la préparation de polyisocyanates contenant des groupes iminooxadiazinedione, dans lequel au moins un diisocyanate et/ou triisocyanate monomérique est oligomérisé en présence

a) d'au moins un catalyseur,
b) d'au moins un additif (A) doté d'une permittivité relative à une température de 18 °C à 30 °C d'au moins 4,0, mesurée comme indiqué dans la description,
c) éventuellement d'autres additifs différents de A,

1 à 50 % en poids d'additif (A) étant utilisé(s) par rapport à la masse du diisocyanate et/ou du triisocyanate monomérique.

**2.** Procédé selon la revendication 1, **caractérisé en ce que** l'additif (A) est ajouté au diisocyanate et/ou au triisocyanate monomérique avant la mise en contact avec le catalyseur.

**3.** Procédé selon la revendication 1 ou 2, **caractérisé en ce que** des nitriles, des carbonates et/ou des lactones cycliques sont utilisé(e)s en tant qu'additif (A), en particulier choisis dans le groupe comprenant l'acétonitrile, l'adiponitrile, le carbonate d'éthylène, le carbonate de propylène et la gamma-valérolactone.

**4.** Procédé selon l'une quelconque des revendications précédentes, **caractérisé en ce que** 2 à 30 % en poids, particulièrement préférablement 2 à 20 % en poids d'un additif (A) sont utilisés, par rapport à la masse du diisocyanate et/ou du triisocyanate monomérique.

**5.** Procédé selon l'une quelconque des revendications précédentes, **caractérisé en ce que** l'additif (A) présente une permittivité relative à une température de 18 °C à 30 °C d'au moins 8,0, préférablement d'au moins 20,0, particulièrement préférablement d'au moins 30,0 ou d'au moins 35,0.

**6.** Procédé selon l'une quelconque des revendications précédentes, **caractérisé en ce qu'**un diisocyanate aliphatique est utilisé en tant que diisocyanate et/ou triisocyanate monomérique, en particulier le diisocyanate d'hexaméthylène (HDI), le 1,5-diisocyanate de 2-méthylpentane, le 1,6-diisocyanate de 2,4,4-triméthyl-hexane, le 1,6-diisocyanate de 2,2,4-triméthyl-hexane et/ou le 1,8-diisocyanate de 4-isocyanatométhyl-octane, préférablement l'HDI.

**7.** Procédé selon l'une quelconque des revendications précédentes, **caractérisé en ce qu'**un sel de tétraorganylammonium et/ou un sel de tétraorganylphosphonium est utilisé en tant que catalyseur, les anions du sel de tétraorganylammonium et/ou du sel de tétraorganylphosphonium étant en particulier choisis dans le groupe : $RfCR_1R_2COO^-$, Rf représentant un radical perfluoroalkyle linéaire ou ramifié et $R_1$ et $R_2$ représentant indépendamment l'un de l'autre H, des radicaux organyle linéaires ou ramifiés, fluorure ($F^-$), di(hydrogéno)fluorure et/ou poly(hydrogéno)fluorure ($[F^- \times HF]_m$), m possédant une valeur de 0,001 à 20, préférablement 0,1 à 20, particulièrement préférablement 0,5 à 20, tout particulièrement préférablement 0,5 à 5.

**8.** Procédé selon la revendication 7, **caractérisé en ce que** le di (hydrogéno) fluorure et/ou le poly(hydrogéno)fluorure ($[F^- \times HF]_m$) est un fluorure d'ammonium quaternaire, un difluorure d'ammonium, un trifluorure d'ammonium, un polyfluorure d'ammonium supérieur, un fluorure de phosphonium, un difluorure de phosphonium, un trifluorure de phosphonium et/ou un polyfluorure de phosphonium supérieur, de préférence ceux qui peuvent être préparés par mélange de fluorures ou d'hydroxydes d'ammonium ainsi que de phosphonium quaternaires avec des quantités correspondantes de fluorure d'hydrogène éventuellement pré-dissous dans des alcools ou de l'eau.

9. Procédé selon l'une quelconque des revendications précédentes, **caractérisé en ce que** le catalyseur/le mélange de catalyseur est utilisé en une proportion de 1 ppm en moles à 1 % en moles, préférablement 5 ppm en moles à 0,1 % en moles, à chaque fois par rapport à la quantité de diisocyanate et/ou de triisocyanate monomérique.

10. Procédé selon l'une quelconque des revendications précédentes, **caractérisé en ce que** le procédé est réalisé dans une plage de température 0 °C à + 250 °C, préférablement 20 à 180 °C, particulièrement préférablement 40 à 150 °C.

11. Procédé selon l'une quelconque des revendications précédentes, **caractérisé en ce que** l'oligomérisation est interrompue, après que 5 à 80 % en poids du diisocyanate et/ou du triisocyanate monomérique sont transformés, préférablement 10 à 60 % en poids.

12. Procédé selon la revendication 11, **caractérisé en ce que** l'oligomérisation est interrompue par désactivation du catalyseur, en particulier par l'ajout d'un acide ou d'un dérivé d'acide comme le chlorure de benzoyle, d'un ester acide d'acides contenant du phosphore ou du soufre, de ces acides eux-mêmes, une liaison adsorptive du catalyseur et une séparation subséquente par filtration ou des combinaisons correspondantes.

13. Procédé selon la revendication 11 ou 12, **caractérisé en ce que** le monomère n'ayant pas réagi est séparé du mélange réactionnel.

14. Système de réaction pour la préparation de polyisocyanates contenant des groupes iminooxadiazinedione, comprenant au moins un diisocyanate et/ou triisocyanate monomérique, ainsi que

 a) au moins un catalyseur,
 b) au moins un additif (A) doté d'une permittivité relative v à une température de 18 °C à 30 °C d'au moins 4,0, mesurée comme indiqué dans la description,
 c) éventuellement d'autres additifs différents de A,

 1 à 50 % en poids d'additif (A) étant utilisé(s) par rapport à la masse du diisocyanate et/ou du triisocyanate monomérique.

15. Utilisation de composés dotés d'une permittivité relative à une température de 18 °C à 30 °C d'au moins 4,0, mesurée comme indiqué dans la description, en tant qu'additif (A) pour la préparation de polyisocyanate contenant des groupes iminooxadiazinedione par modification catalysée de diisocyanates et/ou de triisocyanates monomériques, 1 à 50 % en poids d'additif (A) étant utilisé(s) par rapport à la masse des diisocyanates et/ou des triisocyanates monomériques.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 962455 A1
- EP 962454 A1
- EP 896009 A1
- EP 798299 A1
- EP 447074 A1
- EP 379914 A1
- EP 339396 A1
- EP 315692 A1
- EP 295926 A1
- EP 235388 A1
- EP 1939171 A1
- EP 896009 A

**In der Beschreibung aufgeführte Nicht-Patentliteratur**

- **H. J. LAAS et al.** *J. Prakt. Chem.,* 1994, vol. 336, 185
- **D. WENDISCH ; H. REIFF ; D. DIETERICH.** *Die Angewandte Makromolekulare Chemie,* 1986, vol. 141, 173-183